# EUROPEAN PATENT SPECIFICATION

(11) **EP 3 939 592 B1**
(45) Date of publication and mention of the grant of the patent: **02.09.2026**
(21) Application number: 20772991.4
(22) Date of filing: 16.03.2020
(51) Int. Cl.: A61K 31/47, A61K 39/395, A61K 35/00, A61P 35/00, A61K 45/06, A61K 39/00, C07K 16/28

(54) **COMBINATION OF A FLUORO-SUBSTITUTED LENVATINIB DERIVATIVE AND A PD-1 OR PD-L1 ANTIBODY FOR USE IN THE TREATMENT OF A TUMOR**
KOMBINATION EINES FLUOR-SUBSTITUIERTEN LENVATINIB-DERIVATS UND EINES PD-1- ODER PD-L1-ANTIKÖRPERS ZUR VERWENDUNG BEI DER BEHANDLUNG EINES TUMORS
COMBINAISON D'UN DÉRIVÉ DE LENVATINIB FLUOROSUBSTITUÉ ET D'UN ANTICORPS PD-1 OU PD-L1 POUR UNE UTILISATION DANS LE TRAITEMENT D'UNE TUMEUR

(30) Priority: 15.03.2019 CN 201910198729
(43) Date of publication of application: 19.01.2022
(73) Proprietor: Chongqing Pharmaceutical Industrial Research Institute Co. Ltd., Chongqing 400061 (CN); Yaopharma Co., Ltd., Chongqing 401121 (CN)
(72) Inventor: ZHANG, Yang, Shanghai 200131 (CN); CHEN, Zhengxia, Shanghai 200131 (CN); LI, Jian, Shanghai 200131 (CN); CHEN, Shuhui, Shanghai 200131 (CN)
(74) Representative: ABG Intellectual Property Law, S.L.
(86) International application number: PCT/CN2020/079540
(87) International publication number: WO 2020/187188

(56) References cited:
- WO-A1-2016/140717
- WO-A1-2019/222075
- CN-A- 109 134 365
- CN-A- 109 134 365
- KATO YU ET AL: "Lenvatinib plus anti-PD-1 antibody combination treatment activates CD8+ T cells through reduction of tumor-associated macrophage and activation of the interferon pathway", vol. 14, no. 2, 27 February 2019 (2019-02-27), pages e0212513, XP055790364, Retrieved from the Internet <URL:https://www.ncbi.nlm.nih.gov/pmc/articles/PMC6392299/pdf/pone.0212513.pdf> DOI: 10.1371/journal.pone.0212513
- LI TING-TING ET AL: "Toll-like receptor signaling in colorectal cancer: Carcinogenesis to cancer therapy", WORLD JOURNAL OF GASTROENTEROLOGY, vol. 20, no. 47, 21 December 2014 (2014-12-21), CN, pages 17699 - 17708, XP093029792, ISSN: 1007-9327, DOI: 10.3748/wjg.v20.i47.17699
- KIMURA, T. ET AL.: "Immunomodulatory Activity of Lenvatinib Contributes to Antitumor Activity in the Hepa1-6 Hepatocellular Carcinoma Model", CANCER SCIENCE, vol. 109, 31 December 2018 (2018-12-31), XP055734024, DOI: 20200527150915Y

## Description

This application claims the priority of CN201910198729.7 filed on March 15, 2019.

### FIELD

The present disclosure relates to application of combination of a class of quinoline derivatives with immunomodulators in preparation of antitumor drugs.

### BACKGROUND

Protein tyrosine kinase (PTK) is an enzyme, which phosphorylates tyrosine residues in peptides and proteins, together with ATP being as a substrate. These enzymes are key factors in the regulation of cell signal transduction, such as cell proliferation and differentiation. Specifically, PTK includes receptor tyrosine kinases including hepatocyte growth factors (HGF), platelet derived growth factors (PDGF) and kinases acting in angiogenesis (FGF and VEGF). In addition, it further includes nonreceptor tyrosine kinases including LCK and ABL.

The c-Met protein (also known as hepatocyte growth factor (HGF) receptor) is a transmembrane 190kDa heterodimer with tyrosine kinase activity, and is encoded by c-Met oncogene. It has been showed that HGF/c-Met signaling pathway is confirmed in various cellular responses, including mitogenic activity, proliferative activity, morphogenetic activity and angiogenic activity. Inhibitors of the HGF/c-Met pathway have significant potential for treating cancers.

ABL is a tyrosine kinase encoded by a proto-oncogene, and the activated ABL is able to promote cell proliferation, differentiation and EMT. In haematomas, it is manly activated through gene fusion such as BCR-ABL. In solid tumors, it is manly activated through gene amplification, overexpression and upstream receptor tyrosine kinases, such as PDGFR and EGFR. TNIK is a serine/threonine kinase, which is capable of bonding to β-catenin/TCF in wnt signaling pathway, activating downstream target genes of wnt signaling and promoting tumor growth. MINK is a member of the STE20 protein kinase family, which is highly expressed in the central nervous system, and capable of activating JNK and p38 signaling pathways.

FGFR is a class of biologically active substances, having functions of transmitting biological signals, regulating cell growth, and participating in tissue repair. In recent years, it has been found that a plurality of FGFR family members take important roles in the occurrence and development of tumors. Fibroblast growth factor receptor (FGFR) is a type of receptor protein capable of specifically binding to fibroblast growth factor (FGF), and FGFRs family includes the following types: FGFR1b, FGFR1c, FGFR2b, FGFR2c, FGFR3b, FGFR3c, and FGFR4. Different subtypes of FGFRs will bind with different FGFs, and the bonding of FGFs and FGFRs causes autophosphorylation of a plurality of intracellular tyrosine residues, and phosphorylated FGFRs activate downstream signaling pathways including MEK/MAPK, PLCy/PKC, PI3K/AKT, STATS, ect. Among them, FGFR4 is highly expressed in liver cancer, colon cancer, gastric cancer, esophageal cancer, and testicular cancer, while FGF19, which specifically binds to FGFR4, is highly expressed in human colon cancer, liver cancer and lung cancer cells, and thus signal abnormalities of specific binding of FGFR4 and FGF19 is an important factor for various tumor occurrence and metastasis.

Vascular endothelial growth factor (VEGF) and platelet derived growth factor (PDGF) play an important role in tumor angiogenesis. They bond with their receptors VEGFR or PDGFR, and transmit signals to the intracellular area, and further generate phosphorylated dimers which activates this signaling pathway, and transmit energy downstream, thereby resulting in uncontrolled tumor cell growth, metastasis, and proliferation.

For targets, such as ABL, C-Met, TNIK, FGFR₁-4, VEGFR (FLT1, KDR, FLT4) shown above, considering from the view point of molecular mechanism in the treatment of tumor cells by PDGFR, targets can produce synergistic complementarity among themselves, reducing escape of tumor cells, reducing drug resistance, and improving therapeutic effects, therefore drugs acting on these targets at the same time are much expected.

Kao Yu et al. discloses the use of combination of lenvatinib with anti-PD-1 Ab in the treatment of colon cancer in a mouse model, in a research article, PLOS ONE, Vol. 14, No. 2, 27 February 2019, with a title of "Lenvatinib plus anti-PD-1 antibody combination treatment activates CD8⁺ T cells through reduction of tumor-associated macrophage and activation of the interferon pathway". It is reported that anti-tumor activity of combination of lenvatinib with anti-PD-1 was greater than that of either single treatment.

### SUMMARY

The present disclosure provides a combination of a compound of Formula (I): 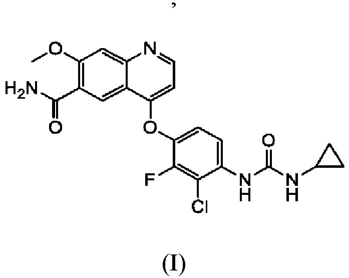 or a pharmaceutically acceptable salt thereof, with an immunomodulator selected from a PD-1 antibody and a PDL-1 antibody, for use in treating a tumor.

In a particular embodiment of the present disclosure, the pharmaceutically acceptable salt of the compound of formula (I) has a structure of:

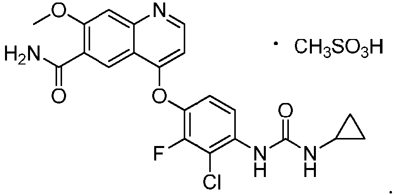

In some embodiments of the present disclosure, the above-mentioned PD-1 antibody is selected from pembrolizumab, nivolumab, sintilimab, toripalimab, RMP1-14, camrelizumab, tislelizumab and Cemiplimab.

In some embodiments of the present disclosure, the above-mentioned PDL-1 antibody is selected from Avelumab, atezolizumab and Durvalumab.

In some embodiments of the present disclosure, the above-mentioned compound of Formula (I) or pharmaceutically acceptable salt thereof and the immunomodulator are administered separately.

In some embodiments of the present disclosure, the above-mentioned compound of Formula (I) or pharmaceutically acceptable salt thereof and the immunomodulator are administered simultaneously.

In some embodiments of the present disclosure, the compound of Formula (I) or a pharmaceutically acceptable salt thereof is administered, after the above-mentioned immunomodulator.

In some embodiments of the present disclosure, the compound of Formula (I) or a pharmaceutically acceptable salt thereof is administered, before the above-mentioned immunomodulator is administered.

In some embodiments of the present disclosure, the above-mentioned compound of Formula (I) or pharmaceutically acceptable salt thereof is administered before 24 hours (1 day), 2 days, 3 days, 4 days or 5 days of administration of the immunomodulator.

In some embodiments of the present disclosure, compared with single compound of Formula (I) or pharmaceutically acceptable salt thereof, or single immunomodulator, the above-mentioned compound of Formula (I) or pharmaceutically acceptable salt thereof and the above-mentioned immunomodulator treat a tumor in a synergistic way.

The present disclosure further provides a composition comprising: a compound represented by Formula (I) or an isomer or a pharmaceutically acceptable salt thereof, and an immunomodulator being a PD-1 antibody.

In some embodiments of the present disclosure, the above-mentioned composition is for use in treating a tumor .

In some embodiments of the present disclosure, the above-mentioned tumor is selected from hepatocellular carcinoma, renal cell carcinoma, endometrial carcinoma, gastric cancer, bile duct cancer, non-small cell lung cancer, melanoma, urinary epithelial cell carcinoma, malignant glioma, esophageal cancer, pancreatic cancer, breast cancer, bowel cancer, lymphadenoma, cervical cancer and brain cancer.

### Definition and Description

Unless otherwise stated, the following terms and phrases used herein are intended to have the following meanings. A specific term or phrase for which no special definition is provided should not be considered uncertain or unclear, but should be understood in its ordinary meaning. When a trade name appears herein, it is meant to refer to the corresponding commodity or the active ingredient thereof.

The term "pharmaceutically acceptable" used herein refers to those compounds, materials, compositions and/or dosage forms that within the scope of reliable medical judgment are suitable for contacting with human and animal tissues, without excessive toxicity, irritation, allergic reactions or other problems or complications, and that are commensurate with a reasonable benefit/risk ratio.

The term "pharmaceutically acceptable salt" refers to a salt of the compound of the present disclosure, which is prepared from a compound with specific substituents discovered in the present disclosure and a relatively non-toxic acid or base. When the compound of the present disclosure contains a relatively acidic functional group, a base addition salt can be obtained by contacting the neutral form of the compound with a sufficient amount of base in a pure solution or a suitable inert solvent. Pharmaceutically acceptable base addition salts include sodium salts, potassium salts, calcium salts, ammonium salts, organic ammonium salts or magnesium salts or similar salts. When the compound of the present disclosure contains a relatively basic functional group, an acid addition salt can be obtained by contacting the neutral form of the compound with a sufficient amount of acid in a pure solution or a suitable inert solvent. Examples of pharmaceutically acceptable acid addition salts include inorganic acid salts, said inorganic acids include, for example, hydrochloric acid, hydrobromic acid, nitric acid, carbonic acid, bicarbonate, phosphate, monohydrogen phosphoric acid, dihydrogen phosphate, sulfuric acid, hydrogen sulfate, hydroiodic acid, phosphorous acid, etc.; as well as organic acid salts, wherein the organic acid includes, for example, acetic acid, propionic acid, isobutyric acid, maleic acid, malonic acid, benzoic acid, succinic acid, octanedioic acid, fumaric acid, lactic acid, mandelic acid, phthalic acid, benzenesulfonic acid, *p*-toluene sulfonic acid, citric acid, tartaric acid and methanesulfonic acid, etc.; and also include salts of amino acids (such as arginine, etc.), and salts of organic acids such as glucuronic acid. Certain specific compounds of the present disclosure contain both basic and acidic functional groups, and thus can be converted into any base or acid addition salt.

The pharmaceutically acceptable salt of the present disclosure can be synthesized from the parent compound containing an acidic or basic functional group by conventional chemical methods. Generally, such salts are prepared by reacting these compounds in free acid or base form with stoichiometric amounts of appropriate bases or acids in water or organic solvents or a mixture of both.

The compound of the present disclosure may exist in specific geometric or isomeric form. The present disclosure contemplates such compounds, including *cis* and *trans* isomers, (-)- and (+)-enantiomers, (*R*)- and (*S*)-enantiomers, diastereomers, (*D*)-isomers, (*L*)-isomers, and the racemic mixture and other mixtures thereof, such as enantiomers or diastereomeric enriched mixtures, which are all within the scope of the present disclosure. All these stereoisomers (as opposed to structural isomers) and their mixtures are included in the scope of the present disclosure.

The optically active (*R*)- and (*S*)-isomers and *D* and *L* isomers can be prepared by chiral synthesis or chiral reagents or other conventional techniques. In order to obtain an enantiomer of a compound of the present disclosure, it can be prepared by asymmetric synthesis or derivatization with chiral auxiliary agents, in which the resulting mixture of diastereomers is separated, and the auxiliary group is cleaved to provide the pure desired enantiomer. Alternatively, when the molecule contains a basic functional group (such as an amino group) or an acidic functional group (such as a carboxyl group), it can form diastereomeric salts with appropriate optically active acids or bases, and then diastereomers can be resolved by conventional methods known in the art to recover the pure enantiomers. In addition, the separation of enantiomers and diastereomers is usually accomplished through the use of chromatography, which employs a chiral stationary phase and is optionally combined with chemical derivatization (for example, a carbamate is generated from an amine).

The compound of the present disclosure may contain unnatural proportions of atomic isotopes on one or more of the atoms constituting the compound. For example, compounds can be labeled with radioisotopes, such as tritium (³H), iodine-125 (¹²⁵I) or C-14 (¹⁴C). For another example, deuterated drugs can be formed by replacing hydrogen with deuterium. The bond between deuterium and carbon is stronger than that of ordinary hydrogen and carbon. Compared with non-deuterated drugs, deuterated drugs have reduced toxic side effects and increased drug stability, enhanced efficacy, extended biological half-life and other advantages. All changes in the isotopic composition of the compounds of the present disclosure, whether radioactive or not, are included in the scope of the present disclosure. "Optional" or "optionally" means that the event or condition described later may but not necessarily occur, and the description includes both the situation where the event or condition occurs and the situation where the event or condition does not occur.

The compound of the present disclosure can be prepared by a variety of synthetic methods well known to those skilled in the art, including the specific embodiments listed below, the embodiments formed by their combination with other chemical synthesis methods, and equivalents or alternatives thereof known to those skilled in the art. Preferred embodiments include but are not limited to the examples of the present disclosure.

The solvent used in the present disclosure is commercially available. The present disclosure uses the following abbreviations: DCM stands for dichloromethane; DMSO stands for dimethyl sulfoxide; EtOAc stands for ethyl acetate; EtOH stands for ethanol; MeOH stands for methanol.

Compounds are named according to common rules for naming compounds in the field or by ChemDraw^{®} software, and commercially available compounds are indicated by supplier catalog names.

**Technical effect:** The compound of the present disclosure exhibited significant anti-tumor activity in combination with PD-1 antibody based in the mouse CT26 tumor model. They significantly enhanced the anti-tumor effect of PD-1 antibody.

### BRIEF DESCRIPTION OF DRAWINGS

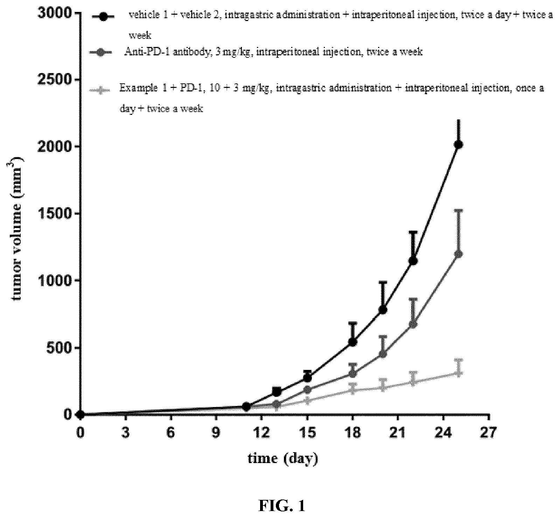

FIG. 1 shows the effect on tumor volumes in the mouse CT26 colon tumor model of Example 1.

### DETAILED DESCRIPTION

The present disclosure has been described in detail herein, and its specific embodiments are also disclosed.

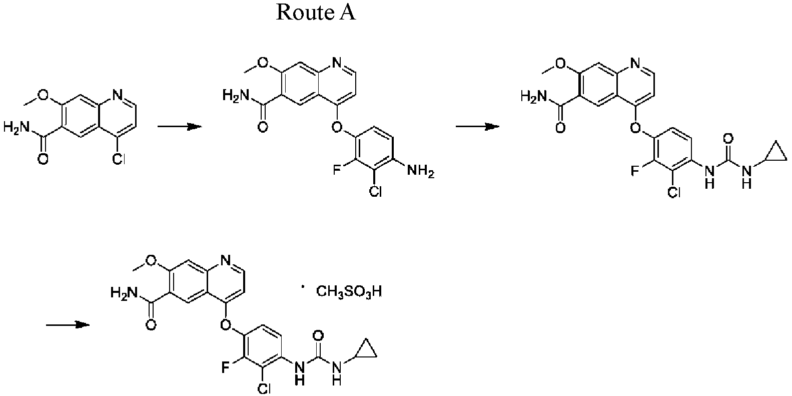

### Example 1 A

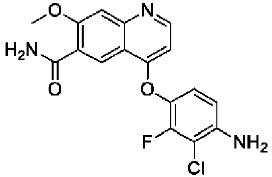

At 20-30°C, 4-chloro-7-methoxyquinoline-6-carboxamide (550.0 g) was added into a reaction kettle. At 20-30°C, DMSO (16.5 L) was added into the reaction kettle. At 20-30°C, 2-fluoro-3-chloro-4-aminophenol was added into the reaction kettle. At 20-35°C, sodium *tert-*butoxide (229 g) was slowly added into the reaction kettle within 10-15 minutes under stirring. The reaction kettle was heated to 96°C (internal temperature) in 1.5 hours. The reaction was stirred at 96-100°C for 6.5 hours, until that no 4-amino-3-chloro-2-fluorophenol was left. The reaction was cooled to 20-30°C. Under stirring, 23.1 L water was slowly added into the reaction solution, in the process of which a dark brown solid precipitated out, as the internal temperature was kept below 40°C. The reaction solution was stirred at 30-40°C for 0.5 hours, cooled to 20-30°C, and filtered. At 20-30°C, the filter cake and 3.5 L water were added into a reaction kettle, stirred at 20-30°C for 0.5 hours, and filtered. At 20-30°C, the filter cake and 4.0 L water were added into a reaction kettle, stirred at 20-30°C for 0.5 hour, and filtered. The filter cake was dried in a vacuum dryer at 40°C for 18 hours (using phosphorus pentoxide as a desiccating agent, and an oil pump to vacuumize). The solid was pulverized to obtain 758 g of off-white solid and dried at 40°C for 18 hours (using phosphorus pentoxide as a desiccating agent, and an oil pump to vacuumize), and Example 1A was obtained.

LCMS (ESI) m/z: 362.0 [M+1]⁺

¹H NMR (400 MHz, DMSO-d₆) δ ppm 8.68 (br s, 2H), 7.82-7.96 (m, 1H), 7.67-7.82 (m, 1H), 7.46-7.59 (m, 1H), 7.12-7.26 (m, 1H), 6.67-6.80 (m, 1H), 6.43-6.58 (m, 1H), 5.84 (s, 2H), 4.04 (s, 3H).

### Example 1B

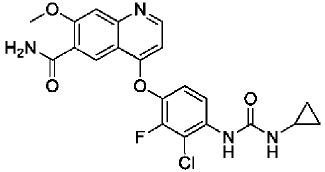

Example 1A (6.05 g) was added into a three-necked flask containing NMP (60 mL). To the reaction system, pyridine (1.32 g), and phenyl chloroformate (5.20 g) were added, and stirred at room temperature (25-30°C) for 1 hour. After the generation of intermediate was complete, cyclopropylamine (2.84 g) was also added to the reaction system. The reaction solution was stirred at room temperature (25-30°C) for 0.5 hours. When the reaction was complete, the reaction system was added with 20 mL ethanol, and then with tap water (500 mL) under stirring, resulting in a solid precipitation, and filtered. The filter cake was dried by rotary evaporation under reduced pressure, to obtain a crude product (a soil yellow solid, 5.26 g). The crude product was purified by a chromatography column (DCM : MeOH = 20/1-10/1), to obtain a product (a soil yellow solid, 3.12 g). To the product, 4 mL anhydrous ethanol was added at normal temperature and stirred for 18 h, and filtered. The filter cake was washed with 1 mL ethanol, and dried under reduced pressure, to obtain Example 1B. From this compound, the corresponded salt was obtained by adding 1 equivalent of hydrochloric acid or sulfuric acid or methanesulfonic acid in acetone or ethanol solution.

LCMS (ESI) m/z: 445.0 [M+1]⁺

¹H NMR (400 MHz, DMSO-d₆) ppm 8.66-8.71 (m, 2H), 8.12-8.20 (m, 2H), 7.72-7.93 (m, 2H), 7.45 (t, J=9.16 Hz,1H), 7.28 (d, J=2.76 Hz, 1H), 6.58 (d, J=5.02Hz, 1H), 4.05 (s, 3H), 2.56-2.64 (m, 1H), 0.38-0.77 (m, 4H).

### Example 1

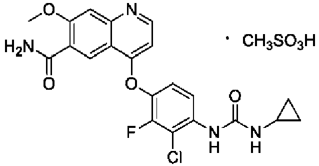

Example 1B (1.5 g, 3.37 mmol) was added to EtOH (45 mL). As the reaction temperature rised to 60°C, CH₃SO₃H (324.07 mg, 3.37 mmol, 240.05 µL) was added dropwise to the reaction solution at this temperature. After the addition was finished, the reaction solution was clear, and allowed to drop to 15-20°C naturally under stirring at this temperature, and stirred for 2 h at this temperature. A lot of brown solid precipitated out, and was filtered. The filter cake was rinsed with anhydrous ethanol (5 mL). The resulting filter cake was dried at 50°C by rotary evaporation under reduced pressure, to obtain Example 1 without purification.

LCMS (ESI) m/z: 445.0 [M+1]⁺

¹H NMR (400MHz, DMSO-d₆) δ ppm 9.02 (d, J=6.53 Hz, 1H), 8.72 (s, 1H), 8.18-8.27 (m, 2H), 7.87-8.03 (m, 2H), 7.65 (s, 1H), 7.53 (t, J=9.03Hz, 1H), 7.32 (br s, 1H), 7.11 (d, J=6.27Hz, 1H), 4.08(s, 3H), 2.55-2.62 (m, 1H), 2.35 (s, 3H), 0.34-0.75 (m, 4H).

### Biological Test Data:

### Experimental example 1: Test on the in vivo anti-tumor activity of the compound of the present disclosure in an animal tumor model

The experimental purposes was to investigate the in *vivo* anti-tumor effect of the combination of the test compound with a mouse anti-PD-1 antibody against the mouse colon cancer in CT26 tumor model.

### Experimental method:

Female Balb/c mice were subcutaneously inoculated with CT26 mouse colon carcinoma cell strain, and randomly grouped based on their body weight, after inoculation. And, they were administered as follows.

To the first group (a control group), the administration was started in the afternoon of the day of vaccination in a way that vehicle 1 (10% DMSO + 10% Solutol + 80% DDH2O) was administered intragastrically twice a day at a dosage of 0.1 mL/10 g body weight each time, and when the tumor had grown to a volume of about 60 mm³ after the inoculation, it started to do administration in a way that vehicle 2 (DPBS) was given twice a week through intraperitoneal injection at a dosage of 0.1 mL/10 g body weight at each time.

To the second group, when the tumor had grown to a volume of about 60 mm³ after the inoculation, it started to do administration in a way that anti-mouse PD-1 antibody (RMP1-14, supplied by BioXcell) was given through intraperitoneal injection twice a week at a dosage of 3 mg/kg body weight at each time.

To the third group: the administration was started in the afternoon of the day of vaccination in a way that Example 1 (co-suspended in 0.5%MC + 0.2%Tween 80) was administered intragastrically once a day at a dosage of 10 mg/kg body weight, and when the tumor had grown to a volume of about 60 mm³ after the inoculation, it started to do administration in a way that anti-mouse PD-1 antibody (RMP1-14, supplied by BioXcell) was given through intraperitoneal injection twice a week at a dosage of 3 mg/kg body weight.

During the experiment, the mice were weighed three times a week, and after the tumor formed, the tumor volume was measured three times a week at each time when the body weight was measured, and calculated following the equation: length ×width² / 2. Tumor proliferation rate was calculated following the equation: Tumor proliferation rate = Tumor volume in the treatment group / Tumor volume in the control group × 100%.

The differences between groups were statistically analyzed by Student's t-test, and p *<* 0.05 was considered to be a significant difference.

### Experimental results:

In the mouse CT26 tumor model (as shown in FIG. 1), the combination of Example 1 with PD-1 antibody exhibited significant anti-tumor activity, as its tumor proliferation rate on day 25 was 15.38% (*p* < 0.01), which was significantly higher than that in the single antibody administrated group (the tumor proliferation rate in the single antibody administrated group was 59.44%). The comparison of tumor volumes on day 25 between the combination group and the single antibody administrated group had significant differences (*p* < 0.05), indicating that Example 1 had significantly enhanced anti-tumor effect on PD-1 antibody in this experiment.

## Claims

1. A combination of a compound of formula 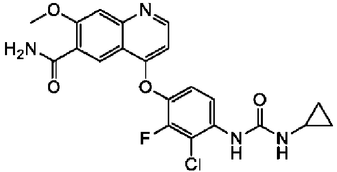 or a pharmaceutically acceptable salt thereof with an immunomodulator selected from a PD-1 antibody and a PDL-1 antibody for use in treating a tumor.

2. The combination for use according to claim 1, wherein the pharmaceutically acceptable salt has a structure of

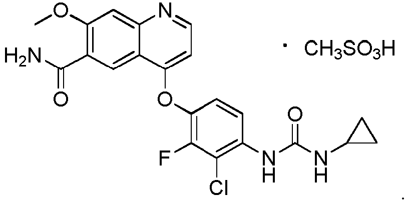

3. The combination for use according to claim 1 or 2, wherein the PD-1 antibody is selected from pembrolizumab, nivolumab, sintilimab, toripalimab, RMP1-14, camrelizumab, tislelizumab and cemiplimab.

4. The combination for use according to claim 1 or 2, wherein the the PDL-1 antibody is selected from avelumab, atezolizumab and durvalumab.

5. The combination for use according to any one of claims 1-4, wherein the compound of formula 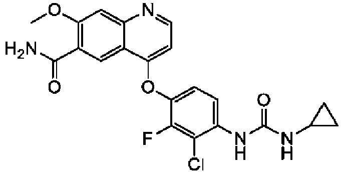 or a pharmaceutically acceptable salt thereof and the immunomodulator are administered separately or simultaneously.

6. The combination for use according to any one of claims 1-4, wherein the compound of formula 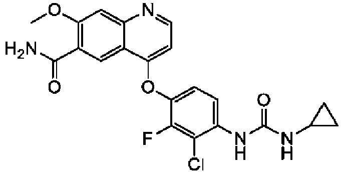 or a pharmaceutically acceptable salt thereof is administered after or before the immunomodulator is administered.

7. The combination for use according to any one of claims 1-4, wherein the compound of formula 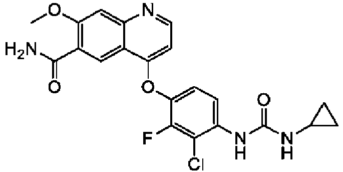 or a pharmaceutically acceptable salt thereof is administered before 24 hours (1 day), 2 days, 3 days, 4 days or 5 days of administration of the immunomodulator.

8. The combination for use according to any one of claims 1-4, wherein the compound of formula 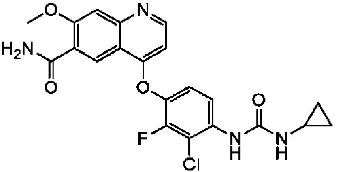 or a pharmaceutically acceptable salt thereof and the immunomodulator treat a tumor in a synergistic way.

9. A composition, comprising:
a compound represented by formula 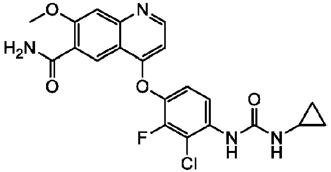 or a pharmaceutically acceptable salt thereof, and
an immunomodulator selected from a PD-1 antibody.

10. The composition according to claim 9, wherein the pharmaceutically acceptable salt has a structure of

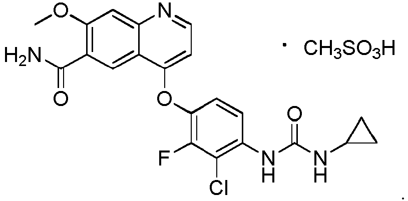

11. The composition according to claim 9 or 10 for use in treating a tumor disease.

12. The combination for use according to claim 1 or the composition for use according to claim 11, wherein the tumor is selected from hepatocellular carcinoma, renal cell carcinoma, endometrial carcinoma, gastric cancer, bile duct cancer, non-small cell lung cancer, melanoma, urinary epithelial cell carcinoma, malignant glioma, esophageal cancer, pancreatic cancer, breast cancer, bowel cancer, lymphadenoma, cervical cancer and brain cancer.

## Patentansprüche

1. Eine Kombination einer Verbindung der Formel 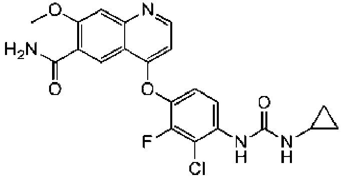 oder eines pharmazeutisch verträglichen Salzes davon mit einem Immunmodulator, der ausgewählt ist aus einem PD-1-Antikörper und einem PDL-1-Antikörper, zur Verwendung bei der Behandlung eines Tumors.

2. Die Kombination zur Verwendung nach Anspruch 1, wobei das pharmazeutisch verträgliche Salz eine Struktur von 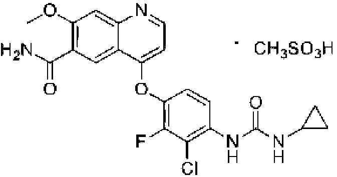 aufweist.

3. Die Kombination zur Verwendung nach Anspruch 1 oder 2, wobei der PD-1-Antikörper ausgewählt ist aus Pembrolizumab, Nivolumab, Sintilimab, Toripalimab, RMP1-14, Camrelizumab, Tislelizumab und Cemiplimab.

4. Die Kombination zur Verwendung nach Anspruch 1 oder 2, wobei der PDL-1-Antikörper ausgewählt ist aus Avelumab, Atezolizumab und Durvalumab.

5. Die Kombination zur Verwendung nach einem der Ansprüche 1-4, wobei die Verbindung der Formel 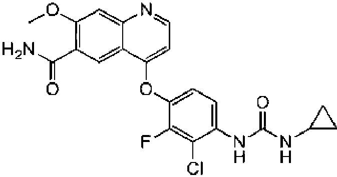 oder ein pharmazeutisch verträgliches Salz davon und der Immunmodulator getrennt oder gleichzeitig verabreicht werden.

6. Die Kombination zur Verwendung nach einem der Ansprüche 1-4, wobei die Verbindung der Formel 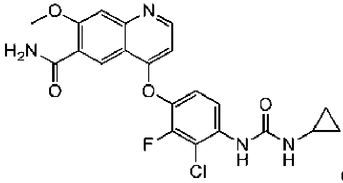 oder ein pharmazeutisch verträgliches Salz davon nach oder vor der Verabreichung des Immunmodulators verabreicht wird.

7. Die Kombination zur Verwendung nach einem der Ansprüche 1-4, wobei die Verbindung der Formel 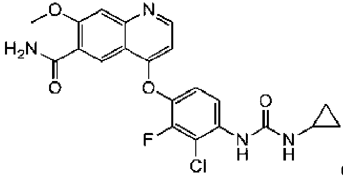 oder ein pharmazeutisch verträgliches Salz davon 24 Stunden (1 Tag), 2 Tage, 3 Tage, 4 Tage oder 5 Tage vor der Verabreichung des Immunmodulators verabreicht wird.

8. Die Kombination zur Verwendung nach einem der Ansprüche 1-4, wobei die Verbindung der Formel 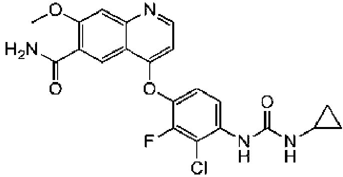 oder ein pharmazeutisch verträgliches Salz davon und der Immunmodulator einen Tumor in synergistischer Weise behandeln.

9. Eine Zusammensetzung, umfassend:
eine Verbindung, die durch die Formel 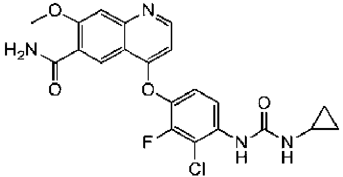 dargestellt ist, oder ein pharmazeutisch verträgliches Salz davon, und
einen Immunmodulator, der aus einem PD-1-Antikörper ausgewählt ist.

10. Die Zusammensetzung nach Anspruch 9, wobei das pharmazeutisch verträgliche Salz eine Struktur von 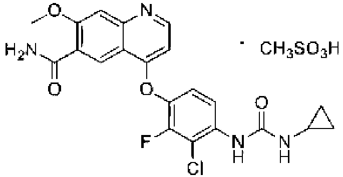 aufweist.

11. Die Zusammensetzung nach Anspruch 9 oder 10 zur Verwendung in der Behandlung einer Tumorerkrankung.

12. Die Kombination zur Verwendung nach Anspruch 1, oder Zusammensetzung zur Verwendung nach Anspruch 11, wobei der Tumor ausgewählt ist aus hepatozellulärem Karzinom, Nierenzellkarzinom, Endometriumkarzinom, Magenkrebs, Gallengangkrebs, nicht-kleinzelligem Lungenkrebs, Melanom, Urothelkarzinom, malignem Gliom, Speiseröhrenkrebs, Bauchspeicheldrüsenkrebs, Brustkrebs, Darmkrebs, Lymphadenom, Gebärmutterhalskrebs und Hirnkrebs.

## Revendications

1. Une combinaison d'un composé de formule 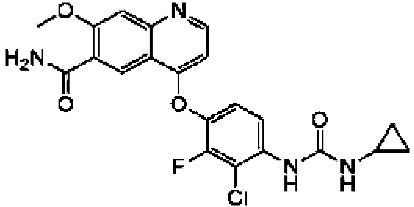 ou
d'un sel pharmaceutiquement acceptable de celui-ci avec un immunomodulateur choisi parmi un anticorps anti-PD-1 et un anticorps anti-PDL-1 pour une utilisation dans le traitement d'une tumeur.

2. La combinaison pour utilisation selon la revendication 1, dans laquelle le sel pharmaceutiquement acceptable a une structure de

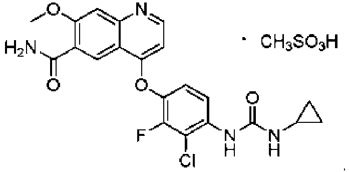

3. La combinaison pour utilisation selon la revendication 1 ou 2, dans laquelle l'anticorps anti-PD-1 est choisi parmi le pembrolizumab, le nivolumab, le sintilimab, le toripalimab, le RMP1-14, le camrelizumab, le tislelizumab et le cemiplimab.

4. La combinaison pour utilisation selon la revendication 1 ou 2, dans laquelle l'anticorps anti-PDL-1 est choisi parmi avélumab, atézolizumab et durvalumab.

5. La combinaison pour utilisation selon l'une quelconque des revendications 1-4, dans laquelle le composé de formule 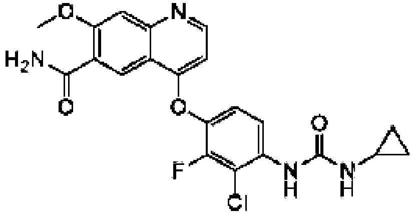 ou
un sel pharmaceutiquement acceptable de celui-ci et l'immunomodulateur sont administrés séparément ou simultanément.

6. La combinaison pour utilisation selon l'une quelconque des revendications 1-4, dans laquelle le composé de formule 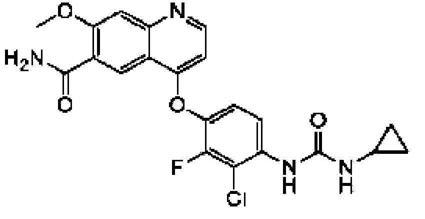 ou un sel pharmaceutiquement acceptable de celui-ci est administré après ou avant que l'immunomodulateur soit administré.

7. La combinaison pour utilisation selon l'une quelconque des revendications 1-4, dans laquelle le composé de formule 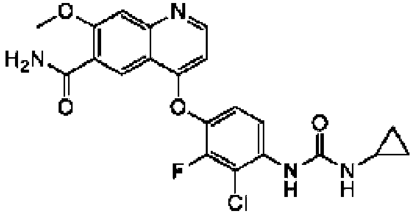 ou un sel pharmaceutiquement acceptable de celui-ci est administré avant 24 heures (1 jour), 2 jours, 3 jours, 4 jours ou 5 jours d'administration de l'immunomodulateur.

8. La combinaison pour l'utilisation selon l'une quelconque des revendications 1-4, dans laquelle le composé de formule 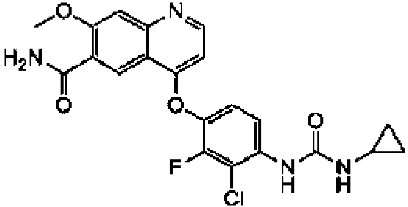 ou un sel pharmaceutiquement acceptable de celui-ci et l'immunomodulateur traitent une tumeur de manière synergique.

9. Une composition, comprenant :
un composé représenté par la formule 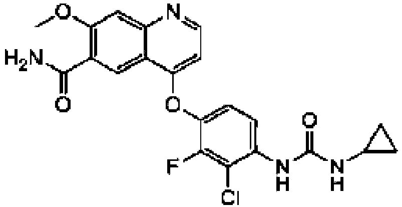 ou
un sel pharmaceutiquement acceptable de celui-ci, et un immunomodulateur choisi parmi un anticorps anti-PD-1.

10. La composition selon la revendication 9, dans laquelle le sel pharmaceutiquement acceptable a une structure de

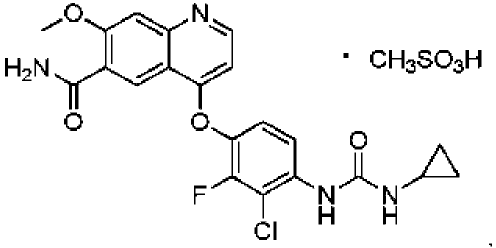

11. La composition selon la revendication 9 ou 10 pour utilisation dans le traitement d'une maladie tumorale.

12. La combinaison pour utilisation selon la revendication 1 ou la composition pour utilisation selon la revendication 11, dans laquelle la tumeur est choisie parmi le carcinome hépatocellulaire, le carcinome à cellules rénales, le carcinome de l'endomètre, le cancer gastrique, le cancer des voies biliaires, le cancer du poumon à cellules non petites, le mélanome, le carcinome des cellules épithéliales urinaires, le gliome malin, le cancer de l'œsophage, le cancer du pancréas, le cancer du sein, le cancer de l'intestin, le lymphadénome, le cancer du col de l'utérus et le cancer du cerveau.
